# EUROPEAN PATENT SPECIFICATION

(11) **EP 2 024 345 B1**
(45) Date of publication and mention of the grant of the patent: **16.12.2009**
(21) Application number: 07729664.8
(22) Date of filing: 30.05.2007
(51) Int. Cl.: C07D 253/06

(54) **METHOD FOR PREPARING LAMOTRIGINE AND ITS INTERMEDIATE 2,3-DICHLOROBENZOYL CHLORIDE**
VERFAHREN ZUR HERSTELLUNG VON LAMOTRIGIN UND DESSEN ZWISCHENPRODUKT 2,3-DICHLORBENZOYLCHLORID
PROCÉDÉ DE PRÉPARATION DE LA LAMOTRIGINE ET SON CHLORURE DE 2,3-DICHLOROBENZOYLE INTERMÉDIAIRE

(30) Priority: 31.05.2006 EP 06290888
(43) Date of publication of application: 18.02.2009
(73) Proprietor: Calaire Chimie SAS, 62104 Calais Cedex (FR)
(72) Inventor: VAN DEYNSE, Dirk, 3582 Koersel (BE); BELMANS, Marc, 3980 Tessenderlo (BE); BOERS, Frank, 2430 Vorst (BE); DUMUR, Michel, 62610 Nielles-les-Ardes (FR); LACONI, Alain, 62137 Coulogne (FR)
(74) Representative: Brants, Johan P.E.
(86) International application number: PCT/EP2007/055251
(87) International publication number: WO 2007/138075

(56) References cited:
- EP-A1- 0 021 121
- US-A- 5 599 980
- US-A- 6 111 101
- BARBOUR ET AL.: "Aromatic Polyfluoro-compounds. Part VIII. Pentafluorobenzaldehyde and Related Pentafluorophenyl Ketones and Carboxylic Acids" JOURNAL OF THE CHEMICAL SOCIETY, 1961, pages 808-817, XP002422333
- SHIPILOV ET AL.: "Selective Hydrolysis of Pentafluorobenzotrichloride" RUSSIAN JOURNAL OF ORGANIC CHEMISTRY, vol. 39, no. 7, 2002, pages 975-978, XP002422334

## Description

### Field of the invention

The invention relates to an improved method for preparing 3,5-diamino-6-(2,3-dichlorophenyl)-1,2,4-triazine, also commonly known as lamotrigine and the intermediate 2,3-dichlorobenzoyl chloride.

### Background

European patent 21121 describes 3,5-diamino-6-(substituted phenyl)-1,2,4-triazines which are active in central nervous system disorders such as psychiatric and neurological disorders, and are also disclosed as anticonvulsants, for example in the treatment of epilepsy. Herein, 3,5-diamino-6-(2,3-dichlorophenyl)-1,2,4-triazine is disclosed.

Lamotrigine can be prepared by the procedures described in, for example, European patent 21121, US patent 4,602,017 or US patent 6,111,101. In these procedures, a condensation reaction of 2,3-dichlorobenzoyl cyanide with an aminoguanidine salt is carried out in a mixture of a large excess of an aqueous mineral acid such as nitric acid or sulfuric acid and a water-miscible organic solvent such as dimethylsulfoxide (DMSO) or acetonitrile. The condensation reaction produces 2-(2,3-dichlorophenyl)-2-(guanidinylamino)acetonitrile, herein referred to as the Schiff base. Further cyclisation of the Schiff base gives crude lamotrigine, which may be further purified by recrystallisation to provide lamotrigine of a pharmaceutically acceptable quality. In these preparations of lamotrigine 2,3-dichlorobenzoyl cyanide is prepared starting from 2,3-dichlorobenzoyl chloride, whereby said 2,3-dichlorobenzoyl chloride is reacted with cuprous cyanide, possibly with potassium iodide, in the presence of an organic solvent, such as e.g. xylene. 2,3-Dichlorobenzoyl chloride can be prepared by reacting 2,3-dichlorobenzoic acid with thionyl chloride in an inert atmosphere.

As lamotrigine has emerged to be one of the more promising anti-epileptic and anticonvulsant agents for treating CNS disorders, its commercial production has assumed greater significance. Despite the several routes known for the synthesis of lamotrigine, the obtained lamotrigine may in some cases be of inadequate purity, such that administration thereof to a patient in need thereof in an appropriate pharmaceutical formulation may cause undesired side-effects. US 5 599 980 discloses a method for preparing lamotrigine starting from 2,3-dichlorobenzoyl chloride.

In addition, methods described for preparing lamotrigine have disadvantages of an environmental and economical type. More in particular, the preparation of 2,3-dichlorobenzoyl chloride involves process reactions which are very cumbersome and laborious and are very costly and often generate large and harmful quantities of waste. Moreover, the obtained 2,3-dichlorobenzoyl chloride is of a moderate and inadequate purity.

In view of the above, it is clear that there remains a need for a lamotrigine synthesis route which is safer, more environmental friendly, efficient, economical and which yields lamotrigine of a higher purity.

Therefore, an objective of the present invention is to provide an improved method for producing lamotrigine, which method would have minimal environmental impact with maximum recycling of side-products and toxic reagents and which would result in the preparation of lamotrigine in high purity.

Another objective is to develop an improved method for preparation of the 2,3-dichlorobenzoylchloride intermediate, and thus to increase the overall efficiency of processes for producing lamotrigine.

### Summary

In a first aspect, the present invention relates to a method for preparing lamotrigine [3,5-diamino-6-(2,3-dichlorophenyl)-1,2,4-triazine].
The present method comprises the steps of:
a) providing 2,3-dichlorotoluene,
b) preparing 2,3-dichlorobenzoyl chloride by photochlorination of said 2,3-dichlorotoluene to produce 2,3-dichlorobenzotrichloride and hydrolyzing said 2,3-dichlorobenzotrichloride to produce 2,3-dichlorobenzoyl chloride,
c) reacting said 2,3-dichlorobenzoyl chloride with a metal cyanide, preferably cuprous cyanide, to produce 2,3-dichlorobenzoyl cyanide,
d) reacting said 2,3-dichlorobenzoyl cyanide with an aminoguanidine salt in aqueous mineral acid to produce the Schiff base [2-(2,3-dichlorophenyl)-2-(guanidinylamino)acetonitrile],
e) reacting said Schiff base in a suitable solvent, with or without the presence of water, to produce lamotrigine, and
f) further crystallizing and drying said lamotrigine.

In the preparation of medicaments and pharmaceutical compositions it is of utmost importance that active compounds that are incorporated in such medicaments or compositions are of the highest possible purity, i.e. contain as less impurities as possible; since the presence of such impurities in the active compounds may cause serious side-effects in patients to which the compositions or medicaments are administered. In view hereof, it is also of importance that the preparation process of active compounds is adequately controlled and performed in the cleanest possible way. It is therefore also very important that especially the first reactions steps in the preparation process of active compounds are well-controlled and yield intermediates having the lowest possible amounts of impurities, since impurities in the intermediates will be further carried throughout the preparation process and ultimately remain in the final active product.

The present invention provides a solution to such problem in the preparation process of lamotrigine. The present invention provides a new process for the synthesis of high purity 3,5-diamino-6-(2,3-dichlorophenyl)-1,2,4-triazine using 2,3-dichlorobenzoyl chloride as starting material. In contrast to prior art processes, the present process involves the use of 2,3-dichlorotoluene as starting material for the preparation of 2,3-dichlorobenzoyl chloride. Moreover, 2,3-Dichlorobenzoyl chloride that is produced by a method according to the present invention is characterized by a very high degree of purity, thus yielding low amounts of impurities. This is reflected in the fact that 2,3 dichlorobenzoyl chloride is obtained in accordance with the present method containing less than 0.1%, and for instance less than 0.08%; 0.07%; 0.06%, or even less than 0.05% of isomers, such as for instance 3,4-dichlorobenzoyl chloride isomers. Said purer 2,3-dichlorobenzoyl chloride is therefore an ideal intermediate in the preparation of lamotrigine.

Furthermore, the lamotrigine preparation method according to this invention has several advantages in contrast to known processes. The main advantage of the present method is the production of very pure final product in high yield. In accordance with the present invention, lamotrigine is obtained showing a high purity, i.e. lower amounts of impurities. This is reflected in the fact that the lamotrigine is obtained in accordance with the present invention wherein the amount of isomers contained in said lamotrigine is lower then 0.1%, and for instance lower than 0.08%; 0.07%; 0.06%, or even lower than 0.05%.

Further advantages of this method involve the possibility to more easily recycle reactants and to eliminate aggressive, hazardous reagents and the shorter reaction time compared to the known procedures. In a preferred embodiment, the present process allows recycling cuprous salts obtained in step c) of the process to cuprous cyanide which can be re-used in the process. The obtained cuprous salts can be recycled to cuprous cyanide with a metal cyanide, preferably sodium cyanide. In another preferred embodiment, 2,3-dichlorobenzoic acid, which is formed as a side-product in the process step d), can be recycled to 2,3-dichlorobenzoyl chloride. The latter can be re-used in the process. Preferably, 2,3-dichlorobenzoic acid is recycled to 2,3-dichlorobenzoyl chloride using 2,3-dichlorobenzotrichloride.

In addition, another advantage is that in contrast to reactions disclosed in the prior art, 2,3-dichlorobenzoyl cyanide can be prepared in accordance with the present process in the absence of a metal iodide such as e.g. KI or NaI. Carrying out the reaction without metal iodides permits to avoid the formation of complex mixtures of inorganic salts that are difficult to separate.

Also, considerable advantage of this process is that it does not require complicated industrial equipment of expensive structural material.

### Detailed description of the invention

The present invention is directed to a method for preparing lamotrigine [3,5-diamino-6-(2,3-dichlorophenyl)-1,2,4-triazine] which is represented by formula (I),

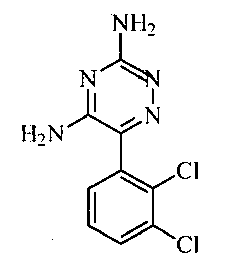

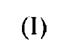

### 2,3-dichlorobenzoyl chloride

In a first step of the present method 2,3-dichlorotoluene is provided and further reacted to produce 2,3-dichlorobenzoyl chloride of formula (IV)

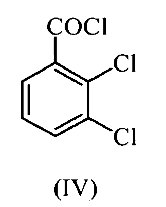

In accordance with the present invention 2,3-dichlorobenzoyl chloride is prepared by photochlorination of said 2,3-dichlorotoluene to produce 2,3-dichlorobenzotrichloride followed by hydrolyzing 2,3-dichlorobenzotrichloride to produce 2,3-dichlorobenzoyl chloride.

The photochlorination reaction is preferably carried out in a UV reactor. In a preferred embodiment the photochlorination reaction is performed using high pressure mercury UV lamps with a power between 2 and 40 kW and a temperature range from 60 to 140 °C, preferably 80 to 120 °C and more preferably 90 to 110 °C. UV-lamps may be doped with Cd, Tl, Ga, In, Fe, Pb or combinations thereof. In a preferred embodiment. UV-lamps are doped with Cd and/or Pb. Doping of the UV lamps increases the selectivity and efficacy of the photochlorination reaction. Using UV lamps further has the advantage that transfer of energy to the compound is done in a more controlled way which does not necessitate the use of a (further) catalyst or radical initiator. The present method for making 2,3-dichlorobenzoyl chloride avoids the need to use a radical initiator, such as e.g. AIBN (azobisisobutyronitrile). Advantageously, therefore also no rest products of such catalysts remain in the prepared 2,3-dichlorobenzoyl chloride which would need to be separated. In accordance with the present process, also other types of UV lamps may be used, e.g. LED lamps.

In another preferred embodiment, the flow of chlorine applied during the photochlorination reaction is linked with the conversion of the starting material and varies between 100 % and 20 % of the initial flow, this way minimizing formation of by-products and impurities.

Preferably, the photochlorination reaction produces 2,3-dichlorobenzotrichloride at a yield of more than 80 %; and preferably more than 85 %, more than 87 %, more than 90 %, more than 92 % and even more than 95 %. In a further preferred embodiment, the hydrolysis reaction is carried out at temperatures which are comprised between 100 and 180 °C, preferably between 120 and 160 °C, in the presence of a suitable catalyst such as metal oxides or metal chlorides, e.g. zinc chloride. Preferably, the hydrolysis reaction produces 2,3-dichlorobenzoyl chloride at a yield of more than 50%; and preferably more than 75 %, more than 90 %, and even more than 95 %.

In addition, in accordance with the above-indicated method 2,3-dichlorobenzoyl chloride is obtained having a very high degree of purity, preferably containing less than 0.1%, and more preferably less than 0.08%; 0.07%; 0.06%, or even less than 0.05% of isomers, e.g. 3,4-dichlorobenzoyl chloride. The present invention therefore also relates to 2,3-dichlorobenzoyl chloride which is obtained by carrying out the above-disclosed method.

### 2,3-dichlorobenzoyl cyanide

A following step in the preparation of lamotrigine comprises reacting 2,3-dichlorobenzoyl chloride obtained in accordance with the method as disclosed above, with a metal cyanide to produce 2,3-dichlorobenzoyl cyanide of formula (III)

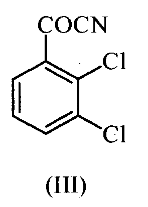

The present reaction may be carried out using CuCN, fresh or recovered as described below.

In a preferred embodiment, the present reaction is carried out at temperatures which are comprised between 140 and 180 °C, preferably during 4 to 10 hours.

In contrast to reactions disclosed in the prior art, the present reaction can also be carried out with recycled CuCN, even in the absence of a solvent and in the absence of an alkali metal iodide such as e.g. KI or NaI, preferably without KI or NaI. The applicant has shown that carrying out the present reaction with recycled CuCN not only reduces production costs, but also advantageously prevents the formation of toxic inorganic waste salts such as e.g. CuCl. In contrast, in the presence of an alkali metal iodide, complex mixtures of inorganic salts are obtained that are difficult to separate. A further important advantage thereof is that the metal cyanide, preferably CuCN, applied in this reaction step can be easily recycled. More in particular, in a preferred embodiment, the inorganic salts, e.g. cuprous salts, which are formed as side-product in this reaction step, are treated with NaCN to produce the CuCN. This recycling step is therefore not only a serious improvement with respect to the cost price of the preparation method, but also with respect to toxic waste destruction/reduction, and environmental problems.

In a further preferred embodiment, after filtration of the salts, the present reaction step further comprises crystallization of the obtained 2,3-dichlorobenzoyl cyanide from a solvent such as n-hexane, n-pentane, n-heptane or petroleum ether.

### 2-(2,3-dichlorophenyl)-2-(guanidinylamino)acetonitrile

Yet a following step in the preparation of lamotrigine comprises reacting 2,3-dichlorobenzoyl cyanide with an aminoguanidine salt in aqueous mineral acid to produce the Schiff base [2-(2,3-dichlorophenyl)-2-(guanidinylamino)acetonitrile] of formula (II)

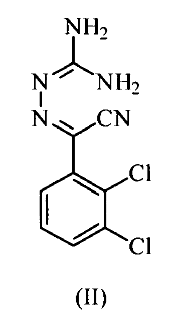

According to the process of the present invention, the condensation reaction of the aminoguanidine salt with 2,3-dichlorobenzoyl cyanide to produce the Schiff base is carried out in an aqueous mineral acid, preferably in the absence of any organic solvent.

The aminoguanidine salt is preferably selected from the group comprising bicarbonate, nitrate, sulfate and hydrochloride salts of aminoguanidine, and most preferably is aminoguanidine bicarbonate.

The aqueous mineral acid is preferably selected from the group comprising hydrochloric acid, hydrobromic acid, nitric acid, sulfuric acid and phosphoric acid, or mixtures thereof, and preferably is sulfuric acid. The concentration of the mineral acid is preferably comprised between 35% v/v to 80% v/v, and more preferably comprised between 60% v/v and 80% v/v.

In a preferred embodiment of the present process, 2,3-dichlorobenzoyl cyanide is reacted with 1 to 4 molequivalent, and preferably 1.5 to 3 molequivalent of aminoguanidine salt, preferably aminoguanidine bicarbonate.

In another preferred embodiment, the 2,3-dichlorobenzoyl cyanide is added to a solution of amidoguanidine salt, preferably aminoguanidine bicarbonate, in aqueous mineral acid, preferably sulfuric acid, at a temperature comprised between 20 and 70 °C, and preferably in crystalline form.

The condensation reaction may be carried out at a temperature in the range of 40 to 80 °C, and preferably in the range of 50 to 70 °C. The reaction may be carried out for 4 to 24 hours, preferably for 6 to 10 hours. The reaction mixture is preferably stirred throughout.

The condensation reaction precipitates Schiffbase salt, and the precipitated Schiff base salt preferably is then filtered. In accordance with the present invention, the present method involves the addition to the reaction mixture prior to filtration of an important amount of water, and preferably a volume of water which is equal to 20 to 40 % of the reaction mixture, facilitating filtration of the Schiff base salt. The filtrate can be used for adjusting the strength of the acid to the desired concentration in a next batch. In this manner, part of the filtrate can be recycled without affecting the quality and yield of the Schiff base. It will be appreciated that the main objective of recycling the aqueous acidic filtrate is to minimize the environmental impact of the process.

The precipitate, comprising Schiff base salt is washed, preferably with water, and then resuspended in an alkaline aqueous solution giving a final pH of about 9 to 10. Aqueous solution of carbonate or hydroxide of an alkali metal such as sodium or potassium can be used or ammonium hydroxide, preferably sodium hydroxide. The liberated base may be filtered and dried, for example at 50 to 70 °C, to obtain the dried free Schiff base.

Preferably, the Schiff base obtained in this reaction step is isolated at a yield of more than 70 % and preferably more than 80 %.

The alkaline filtrate further comprises the sodium or potassium salt of 2,3-dichlorobenzoic acid, which is formed as side-product in the reaction. Acidifying this filtrate precipitates 2,3-dichlorobenzoic acid that can be isolated by means of filtration and recycled into the process as described below. The applicant has shown that starting from 2,3-dichlorobenzoic acid -which is an unavoidable side product in the total process- 2,3-dichlorobenzoyl chloride can be recycled. In a preferred embodiment, the invention therefore relates to a method, wherein 2,3-dichlorobenzoyl chloride is recycled from the present reaction step by reacting dried 2,3-dichlorobenzoic acid-with 2,3-dichlorobenzotrichloride. This acid can be added to the 2,3-dichlorobenzotrichloride in the hydrolysis reactor prior to dosing of water. Recycling of this 2,3-dichlorobenzoic acid has been proven not to affect quality of the 2,3-dichlorobenzoyl chloride obtained. This recycling step is therefore not only a serious improvement with respect to the cost price of the preparation method, but also with respect to organic waste destruction/reduction, and environmental problems.

### Lamotrigine

A subsequent step in the preparation of lamotrigine comprises reacting the Schiff base obtained as disclosed above in a suitable solvent, with or without the presence of water, to produce lamotrigine. The obtained lamotrigine may be further crystallized and dried. Preferably the Schiff base is cyclised in organic solvent, with or without the presence of water, to provide lamotrigine of a pharmaceutically acceptable quality.

The organic solvent used for the cyclisation step according to the present invention is a water-miscible or a water-soluble solvent, for instance an alcohol selected from the group comprising from methanol, ethanol, isopropyl alcohol, n-propanol, n-butanol, tert-butanol and diethylene glycol, or a solvent such as acetonitrile, or any mixtures thereof. Preferably, the organic solvent is an alcohol, most preferably n-propanol.

The cyclisation step is preferably carried out at a temperature in the range from 70 to 110 °C, preferably from 80 to 90 °C. The reaction may be conducted over a period from preferably 20 min to 300 min, more preferably 30 min to 180 min. The reaction mixture is preferably stirred throughout. At the end of the reaction, a clear solution is obtained. According to the present invention, activated carbon is then added to the clear solution, the solution is stirred and the hot solution is filtered. The filtrate is further cooled, e.g. to 40 to 6 °C, to provide crystalline lamotrigine.

In a preferred embodiment, the crystalline lamotrigine obtained as explained above may be re-crystallized and then further dried.

### Examples

### Example 1: Synthesis of 2,3-dichlorobenzotrichloride

A UV photochlorination reactor was loaded with 245 g of 2,3-dichlorotoluene (1.52 moles) and 1225 g of CCl₄. The reactor was heated to 75 °C and chlorine was introduced at a flow of 190 g/h. At the end of the reaction, the flow is decreased to minimize break-through of chlorine. The crude mixture was analyzed showing a content of 2,3-dichlorobenzal chloride < 0.2 %. The solvent CCl₄ was distilled from the reaction mixture at 600 to 800 mbar. The crude product was then let to distil at 13 mbar and 128 °C giving 334.2 g of 2,3-dichlorobenzotrichloride with a GC assay of 98.2 %. Yield was 81.6 %.

### Example 2: Synthesis of 2,3-dichlorobenzoyl chloride

A reactor was loaded with 168.9 g of 2,3-dichlorobenzotrichloride (assay 98.9 %, 0.63 moles). The product was heated to 100 °C and a catalytic amount of ZnCl₂ (0.2 g) was added. The mixture was further heated to 160 °C. To the reactor was added dropwise 12.0 g of water while keeping temperature at 160 °C. Addition time was 7 hours. The crude product was then let to distil giving 120.5 g of 2,3-dichlorobenzoyl chloride with a GC assay of 99.7 %. Yield was 91.0 %.

### Example 3: Synthesis of 2,3-dichlorobenzotrichloride

A UV photochlorination reactor was loaded with 220.5 g of 2,3-dichlorotoluene (1.37 moles) and 1584 g of CCl₄. The reactor was heated to 75 °C and chlorine was introduced at a flow of 190 g/h. At the end of the reaction, the flow was decreased to minimize break-through of chlorine. The crude mixture was analyzed showing a content of 2,3-dichlorobenzal chloride < 0.2 %. The solvent was distilled from the reaction mixture (1808 g) giving 1351 g of CCl₄. The crude 2,3-dichlorobenzotrichloride (366.5 g) had a GC assay of 96.0 %. Yield was 97.1 %.

### Example 4: Synthesis of 2,3-dichlorobenzoyl chloride

A reactor was loaded with 366.5 g of 2,3-dichlorobenzotrichloride (assay 96.0 %, 1.33 moles) and 1 g of ZnCl₂. The mixture was heated to 160-165 °C. To the reactor was added dropwise 24.0 g of water while keeping temperature at 160-165 °C. Addition time was 1 hour. The crude product was then let to distil giving 247.9 g of 2,3-dichlorobenzoyl chloride with a GC assay of 98.9 %. Residue was 23.0 g. Yield was 88.0 %.

### Example 5: Synthesis of 2,3-dichlorobenzotrichloride

A UV photochlorination reactor was loaded with 1042 g of 2,3-dichlorotoluene (6.47 moles). The reactor was heated to 100 °C and chlorine was introduced at a flow of 370 g/h. At the end of the reaction, the flow was decreased to 40 g/h to minimize break-through of chlorine. The crude mixture was analysed showing a content of 2,3-dichlorobenzal chloride of 0.3 %. The 2,3-dichlorobenzotrichloride (1675.5 g) was obtained with a GC assay of 97.2 %. Yield was 95.2 %.

### Example 6: Synthesis of 2,3-dichlorobenzoyl cyanide

A reactor was loaded with 350.0 g of 2,3-dichlorobenzoyl chloride (1.67 moles) and 200.0 g of cuprous cyanide. The mixture was heated to 160-165 °C and stirred at this temperature for 7 hours. The mixture was cooled to 85 °C and 1200 ml of toluene was added to the mixture. The mixture was stirred for 1 hour at 60 °C, cooled to 15 °C and the inorganic salts were filtrated. The solvent toluene was distilled from the filtrate at 55 °C under reduced pressure. The crude product was crystallised from petroleum ether giving 323.3 g of 2,3-dichlorobenzoyl cyanide with an assay of 97.4 %. Yield was 94.2 %.

### Example 7: Recuperation of cuprous cyanide

A reactor was loaded with 300 g of water and 167.2 g of crude CuCl that was filtered during a preparation of 2,3-dichlorobenzoyl cyanide. The mixture was heated to 100 °C and to the reactor was added dropwise a solution of 63.5 g sodium cyanide (1.30 moles) in 300 g of water. The mixture was stirred for 1 hour at 100 °C, cooled to 20 °C, stirred for 1 hour at 20 °C, filtered and washed with 200 ml of methanol. The filtered cuprous cyanide is dried at 80 °C under reduced pressure. Yield of recovered cuprous cyanide is 94.2 %.

### Example 8: Synthesis of 1-(2,3-dichlorophenyl)-2-(guanidinylamino)acetonitrile

A 3L reactor was loaded with 982 g of water. To the reactor was added 1808 g of H₂SO₄ keeping temperature below 90 °C. The solution was cooled down to 25 °C. To the reactor was added 272.1 g of aminoguanidine bicarbonate (2.0 moles) in small portions to control CO₂ evolution. The mixture was stirred for 30 minutes. Then 200.0 g of 2,3-dichlorobenzoyl cyanide (1.0 mole) was added to the aminoguanidine solution. The mixture was heated gently to 60 °C and stirred for 6 hours at 60 °C. The mixture was cooled down to 20 °C and 982 g of water was slowly added keeping temperature below 24 °C. Addition time was 30 min. The precipitate was filtered and washed three times with 500 g portions of water.

A 3L reactor was loaded with 840 g of water and 45 g of NaOH. The content was stirred at 25 °C. To the flask was added portionwise the wet crude filtered cake. The suspension was heated to 60 °C, stirred for 1 hour at 60 °C and filtered while warm. The precipitate was washed three times with 400 g portions of water. The wet cake (496.0 g) was dried at 70 °C and P < 50 mbar, giving 198.2 g of 2-(2,3-dichlorophenyl)-2-(guanidinylamino)acetonitrile with HPLC assay of 99.2 %. Yield was 76.8 %.

### Example 9: Synthesis of 2-(2,3-dichlorophenyl)-2-(guanidinylamino)acetonitrile

A 3L reactor was loaded with 982 g of water. To the reactor was added 1808 g of H₂SO₄ keeping temperature below 90 °C. The solution was cooled down to 25 °C. To the reactor was added 408.4 g of aminoguanidine bicarbonate (3.0 moles) in small portions to control CO₂ evolution. The mixture was stirred for 30 minutes. Then 200.0 g of 2,3-dichlorobenzoyl cyanide (1.0 mole) was added to the aminoguanidine solution. The mixture was heated gently to 60 °C and stirred for 6 hours at 60 °C. The mixture was cooled down to 20 °C and 982 g of water was slowly added keeping temperature below 24 °C. Addition time was 30 min. The precipitate was filtered and washed three times with 500 g portions of water.

A 3L reactor was loaded with 840 g of water and 40 g of NaOH. The content was stirred at 25 °C. To the flask was added portionwise the wet crude filtered cake. The suspension was heated to 60 °C, stirred for 1 hour at 60 °C and filtered while warm. The precipitate was washed three times with 400 g portions of water. The wet cake was dried at 70 °C and P < 50 mbar, giving 221.8 g of 2-(2,3-dichlorophenyl)-2-(guanidinylamino)acetonitrile with HPLC assay of 98.9 %. Yield was 85.6 %.

### Example 10: Synthesis of 2,3-dichlorobenzoyl chloride with recovered 2,3-dichlorobenzoic acid

The alkaline filtrates of example 8 were combined and treated with concentrated HCl until having a suspension at pH 1-2. The precipitate was filtered, washed with water and dried at 60 °C and reduced pressure to give 22.9 g of 2,3-dichlorobenzoic acid. A reactor was loaded with 352.3 g of 2,3-dichlorobenzotrichloride (assay 96.4 %, 1.28 moles) and 1 g of ZnCl₂. The mixture was heated to 160-165 °C. To the reactor was added portionwise the 22.9 g of 2,3-dichlorobenzoic acid followed by dropwise addition of 21.0 g of water while keeping temperature at 160-165 °C. The crude product was then let to distil giving 261.9 g of 2,3-dichlorobenzoyl chloride with a GC assay of 99.1 %. Yield was 88.5 %.

### Example 11: Synthesis of Lamotrigine

A 10L reactor was loaded with 3521 g of *n*-propanol, 1200 g of water and 400.0 g of 2-(2,3-dichlorophenyl)-2-(guanidinylamino)acetonitrile (1.56 moles). The suspension was stirred and gently heated to 87 °C. Stirring was continued for 2 h 10 min at 87 °C until reaction was complete. To the reactor was added 30 g of charcoal and stirring at 87 °C was continued for another 40 min. The charcoal was filtered warm over a glass filter and was washed with 100 g of *n*-propanol. The warm filtrate was stirred well and gently let to cool down to 40 °C. When reaching 40 °C, external cooling was applied and the mixture was gradually cooled down from 40 °C to 8 °C in 6 hours. The mixture was stirred at 8 °C overnight. The precipitate was filtered and washed three times with 250 g portions of *n*-propanol. The wet precipitate was dried for 1.5 h at 50 °C and 10 mbar, followed by 2 h at 100 °C and 7 mbar, giving 352.9 g of Lamotrigine (HPLC assay 99.9 %). Yield was 88.2 %.

### Example 12: Recrystallisation of Lamotrigine

A 10L reactor was loaded with 1100 g of *n*-propanol, 377 g of water and 125.2 g of Lamotrigine crude (0.49 moles). The suspension was stirred and gently heated to 85 °C. To the reactor was added 14 g of charcoal and stirring at 85 °C was continued for 30 min. The charcoal was filtered warm over a glass filter and was washed with 100 g of *n*-propanol. The warm filtrate was stirred well and gently let to cool down to 40 °C. When reaching 40 °C, external cooling was applied and the mixture was gradually cooled down from 40 °C to 11 °C in 6 hours. The mixture was stirred at 11 °C overnight followed by 2 h at 6 °C. The precipitate was filtered and washed three times with 80 g portions of *n*-propanol. The wet precipitate was dried for 1 h at 40 °C and 10 mbar, followed by 2 h at 110 °C and 10 mbar, giving 111.4 g of Lamotrigine (HPLC assay 99.9 %). Yield was 89.0 %.

### Example 13: Synthesis of Lamotrigine

A 10L reactor was loaded with 6000 g of *n*-propanol and 400.0 g of 2-(2,3-dichlorophenyl)-2-(guanidinylamino)acetonitrile (1.56 moles). The suspension was stirred and gently heated to 90 °C. Stirring was continued for 2 h 45 min at 90 °C until reaction was complete. To the reactor was added 30 g of charcoal and stirring at 87 °C was continued for another 30 min. The charcoal was filtered warm over a glass filter and was washed with 100 g of *n*-propanol. The warm filtrate was stirred well and gently let to cool down to 40 °C. When reaching 40 °C, external cooling was applied and the mixture was gradually cooled down from 40 °C to 8 °C in 6 hours. The mixture was stirred at 8 °C overnight. The precipitate was filtered and washed three times with 185 g portions of *n*-propanol. The wet precipitate was dried for 1 h at 40 °C and 40 mbar, followed by 16 h at 140 °C and 8 mbar, giving 338.9 g of Lamotrigine (HPLC assay 99.9 %). Yield was 84.7 %.

## Claims

1. Method for preparing lamotrigine [3,5-diamino-6-(2,3-dichlorophenyl)-1,2,4-triazine], comprising the steps of:
a) providing 2,3-dichlorotoluene,
b) preparing 2,3-dichlorobenzoyl chloride by photochlorination of said 2,3-dichlorotoluene to produce 2,3-dichlorobenzotrichloride and hydrolyzing said 2,3-dichlorobenzotrichloride to produce 2,3-dichlorobenzoyl chloride,
c) reacting said 2,3-dichlorobenzoyl chloride with a metal cyanide, preferably cuprous cyanide (CuCN), to produce 2,3-dichlorobenzoyl cyanide,
d) reacting said 2,3-dichlorobenzoyl cyanide with an aminoguanidine salt in aqueous mineral acid to produce the Schiff base [2-(2,3-dichlorophenyl)-2-(guanidinylamino)acetonitrile],
e) reacting said Schiff base in a suitable solvent, with or without the presence of water, to produce lamotrigine, and
f) further crystallizing and drying said lamotrigine.

2. Method according to claim 1, wherein the photochlorination reaction of step b) is performed using high pressure mercury UV lamps with a power between 2 and 40 kW and a temperature range from 60 to 140 °C.

3. Method according to claim 1 or 2, wherein the flow of chlorine applied during the photochlorination reaction is linked with the conversion of the starting material and varies between 100 % and 20 % of the initial flow.

4. Method according to any of claims 1 to 3, wherein the metal cyanide, preferably CuCN, applied in step c) is obtained by reacting inorganic salts that are formed as side-product in step c) with a metal cyanide, preferably NaCN.

5. Method according to any of claims 1 to 4, wherein 2,3-dichlorobenzoyl cyanide is reacted in step d) with 1 to 4 molequivalent, and preferably 1.5 to 3 molequivalent of aminoguanidine salt.

6. Method according to any of claims 1 to 5, wherein the aminoguanidine salt is aminoguanidine bicarbonate, and the aqueous mineral acid is sulfuric acid.

7. Method according to any of claims 1 to 6, wherein 2,3-dichlorobenzoyl chloride is obtained from step d) by reacting 2,3-dichlorobenzoic acid, which is formed as side-product in step d) with 2,3-dichlorobenzotrichloride.

## Patentansprüche

1. Verfahren zur Herstellung von Lamotrigin [3,5-Diamino-6-(2,3-dichlorphenyl)-1,2,4-triazin], welches die folgenden Schritte umfasst:
a) Bereitstellen von 2,3-Dichlortoluol,
b) Herstellen von 2,3-Dichlorbenzoylchlorid durch Photochlorierung des 2,3-Dichlortoluols, um 2,3-Dichlorbenzotrichlorid zu erzeugen, und Hydrolyse des 2,3-Dichlorbenzotrichlorids, um 2,3-Dichlorbenzoylchlorid zu erzeugen,
c) Zur-Reaktion-Bringen des 2,3-Dichlorbenzoylchlorids mit einem Metallcyanid, vorzugsweise Kupfer(I)-cyanid (CuCN), um 2,3-Dichlorbenzoylcyanid zu erzeugen,
d) Zur-Reaktion-Bringen des 2,3-Dichlorbenzoylcyanids mit einem Aminoguanidinsalz in wässriger Mineralsäure, um die Schiff'sche Base [2-(2,3-Dichlorphenyl)-2-(guadinylamino)acetonitril] zu erzeugen,
e) Zur-Reaktion-Bringen der Schiff'schen Base in einem geeigneten Lösungsmittel in Gegenwart oder Abwesenheit von Wasser, um Lamotrigin zu erzeugen, und
f) Auskristallisieren und Trocknen des Lamotrigins.

2. Verfahren nach Anspruch 1, wobei die Photochlorierungsreaktion des Schrittes b) unter Verwendung von Hochdruck-Quecksilberdampf-UV-Lampen mit einer Leistung von 2 bis 40 kW in einem Temperaturbereich von 60 bis 140 °C durchgeführt wird.

3. Verfahren nach Anspruch 1 oder 2, wobei der Chlorfluss während der Photochlorierungsreaktion mit der Umwandlung des Ausgangsmaterials verbunden ist und zwischen 100% und 20% des anfänglichen Flusses variiert.

4. Verfahren nach einem der Ansprüche 1 bis 3, wobei das Metallcyanid, vorzugsweise CuCN, das in Schritt c) angewendet wird, erhalten wird, indem anorganische Salze, die in Schritt c) als Nebenprodukt gebildet werden, mit einem Metallcyanid, vorzugsweise NaCN, zur Reaktion gebracht werden.

5. Verfahren nach einem der Ansprüche 1 bis 4, wobei in Schritt d) 2,3-Dichlorbenzoylcyanid mit 1 bis 4 Moläquivalenten, vorzugsweise 1,5 bis 3 Moläquivalenten, des Aminoguanidinsalzes zur Reaktion gebracht wird.

6. Verfahren nach einem der Ansprüche 1 bis 5, wobei es sich bei dem Aminoguanidinsalz um Aminoguanidinbicarbonat und bei der wässrigen Mineralsäure um Schwefelsäure handelt.

7. Verfahren nach einem der Ansprüche 1 bis 6, wobei 2,3-Dichlorbenzoylchlorid aus Schritt d) erhalten wird, indem 2,3-Dichlorbenzoesäure, welche in Schritt d) als Nebenprodukt gebildet wird, mit 2,3-Dichlorbenzotrichlorid zur Reaktion gebracht wird.

## Revendications

1. Procédé de préparation de lamotrigine [3,5-diamino-6-(2,3-dichlorophényl)-1,2,4-triazine], comprenant les étapes consistant à :
a) fournir du 2,3-dichlorotoluène,
b) préparer du chlorure de 2,3-dichlorobenzoyle par photochloration dudit 2,3-dichlorotoluène pour produire du 2,3-dichlorobenzotrichlorure et hydrolyser ledit 2,3-dichlorobenzotrichlorure pour produire du chlorure de 2,3-dichlorobenzoyle,
c) faire réagir ledit chlorure de 2,3-dichlorobenzoyle avec un cyanure de métal, de préférence du cyanure de cuivre (CuCN), pour produire du cyanure de 2,3-dichlorobenzoyle,
d) faire réagir ledit cyanure de 2,3-dichlorobenzoyle avec un sel d'aminoguanidine dans un acide minéral aqueux pour produire la base de Schiff [2-(2,3-dichlorophényl)-2-(guanidinylamino)acétonitrile],
e) faire réagir ladite base de Schiff dans un solvant approprié, avec ou sans la présence d'eau, pour produire de la lamotrigine, et
f) cristalliser et sécher ladite lamotrigine.

2. Procédé selon la revendication 1, dans lequel la réaction de photochloration de l'étape b) est effectuée en utilisant des lampes UV à mercure à haute pression avec une puissance comprise entre 2 et 40 kW et une plage de température allant de 60 et 140 °C.

3. Procédé selon la revendication 1 ou 2, dans lequel le flux de chlore appliqué pendant la réaction de photochloration est lié à la conversion du matériau de départ et varie entre 100 % et 20 % du flux initial.

4. Procédé selon l'une quelconque des revendications 1 à 3, dans lequel le cyanure de métal, de préférence CuCN, appliqué dans l'étape c), est obtenu en faisant réagir les sels inorganiques qui sont formés en tant que sous-produit dans l'étape c) avec un cyanure de métal, de préférence NaCN.

5. Procédé selon l'une quelconque des revendications 1 à 4, dans lequel le cyanure de 2,3-dichlorobenzoyle est mis à réagir dans l'étape d) avec 1 à 4 équivalents molaires, et de préférence 1,5 à 3 équivalents molaires de sel d'aminoguanidine.

6. Procédé selon l'une quelconque des revendications 1 à 5, dans lequel le sel d'aminoguanidine est le bicarbonate d'aminoguanidine, et l'acide minéral aqueux est l'acide sulfurique.

7. Procédé selon l'une quelconque des revendications 1 à 6, dans lequel le chlorure de 2,3-dichlorobenzoyle est obtenu à partir de l'étape d) en faisant réagir l'acide 2,3-dichlorobenzoïque, qui est formé en tant que sous-produit dans l'étape d) avec le 2,3-dichlorobenzotrichlorure.
